# EUROPEAN PATENT SPECIFICATION

(11) **EP 1 365 748 B1**
(45) Date of publication and mention of the grant of the patent: **09.11.2005**
(21) Application number: 01949819.5
(22) Date of filing: 18.07.2001
(51) Int. Cl.: A61K 9/28, A61K 9/50, A61K 31/404, A61P 25/06

(54) **PARTICULATE COMPOSITION OF ELETRIPTAN HYDROBROMIDE SHOWING A SIGMOIDAL PATTERN OF CONTROLLED RELEASE**
PARTIKULÄRE ZUSAMMENSETZUNG MIT SIGMOIDEM FREISETZUNGSMUSTER ENTHALTEND ELETRIPTAN HYDROBROMID
COMPOSITION PARTICULAIRE D'HYDROBROMURE D'ELETRIPTAN PRESENTANT UN MODELE SIGMOIDE DE LIBERATION CONTROLEE

(30) Priority: 02.08.2000 GB 0018968
(43) Date of publication of application: 03.12.2003
(73) Proprietor: Pfizer Limited, Sandwich, Kent CT13 9NJ (GB); PFIZER INC., New York, N.Y. 10017 (US)
(72) Inventor: DE RASPIDE, Manaud P.F., Pfizer Global Res. & Dev., Sandwich, Kent CT13 9NJ (GB); MACRAE, Ross J., c/o Pfizer Central Research, Sandwich, Kent CT13 9NJ (GB); WALTHER, Mathias, c/o Pfizer Central Research, Sandwich, Kent CT13 9NJ (GB)
(74) Representative: Ruddock, Keith Stephen
(86) International application number: PCT/IB2001/001279
(87) International publication number: WO 2002/009675

(56) References cited:
- WO-A-00/32589
- WO-A-01/03672
- WO-A-01/23377
- WO-A-96/06842
- US-A- 5 112 621

## Description

This invention relates to a particulate composition containing eletriptan, or a pharmaceutically acceptable salt thereof, which is capable of achieving a sigmoidal pattern of controlled drug release and to processes for the preparation of, pharmaceutical formulations containing and the uses of such a composition.

Eletriptan, 3-{[1-methylpyrrolidin-2(R)-yl]methyl}-5-(2-phenylsulfonylethyl)-1H-indole, is disclosed in WO-A-92/06973. A preferred hydrobromide salt of eletriptan is disclosed in WO-A-96/06842 and a preferred hydrobromide salt monohydrate is disclosed in WO-A-00/32589. A pharmaceutical formulation comprising eletriptan hemisulphate and caffeine is disclosed in WO-A-99/01135 and a complex of eletriptan with a cyclodextrin is disclosed in WO-A-01/00243.

Eletriptan is a 5-HT_{1B/1D} receptor agonist and has been shown to be highly effective in the treatment of migraine. More recently, the use of eletriptan in the prevention of migraine recurrence has been disclosed in WO-A-00/06161. Migraine recurrence is a separate condition from migraine itself and can be defined (see WO-A-00/06161) as the return of a moderate or severe migraine headache within 48 hours, especially within 24 hours, of the first dosing with medication.

In certain instances, it is useful to administer eletriptan to a patient in a controlled way over a period of time. In the preventative treatment of migraine recurrence, for example, it is useful to achieve a delayed and/or sustained release of eletriptan that will protect the patient from the return of a moderate or severe migraine headache within a 24 to 48 hour period from initial dosing of drug. Accordingly, it is an object of this invention to provide a well-tolerated pharmaceutical composition of eletriptan, or a pharmaceutically acceptable salt thereof, suitable for oral administration, that will release eletriptan in the gastrointestinal tract of a patient, after an initial delay and/or over a sustained period of time, in a sigmoidal fashion. Since in those patients susceptible to migraine recurrence it will be more convenient to administer sufficient eletriptan by a means to both treat an initial migraine attack and to prevent migraine recurrence, it is a further object of the present invention to provide a dual release pharmaceutical formulation of eletriptan, or a pharmaceutically acceptable salt thereof, containing both sigmoidal type controlled-release and immediate-release forms of the drug.

In order to achieve an oral, controlled release formulation of a drug, capable of achieving a sigmoidal release, comprising a core of the drug coated with a water-insoluble, polymeric membrane, the drug selected must have certain properties. In particular, its aqueous solubility and dissolution characteristics must be such that it both dissolves adequately at the membrane-drug interface and passes at a suitable rate through the membrane when the formulation is hydrated in the gastrointestinal tract, properties that are extremely difficult, if not impossible, to predict or determine in isolation. The idiosyncratic properties of different drugs and their individual salt forms has prevented the general applicability of such technology and made it impossible to predict whether or not a given drug in a given form can be delivered in a sigmoidal controlled release manner. In any event, it is believed that the utility of such controlled-release technology may be restricted to certain highly water-soluble drugs such as diltiazem hydrochloride (see *Journal of Controlled Release,* 1997, **44,** 263-270). US-A-5 112 621 desribes a formulation of diltiazem in which a core of the drug is coated with a mixture comprising ethyl cellulose and an acrylic resin.

Several salts of eletriptan have been identified that have properties which make them particularly suitable for development as drug substances. These include the hydrobromide salt which has low aqueous solubility (4 mg/ml at 20 °C) similar to eletriptan free base (2.5 mg/ml at 20°C), and the hemisulphate salt which has high aqueous solubility (>200 mg/ml at 20°C). It is therefore desirable to provide a controlled release formulation capable of achieving a sigmoidal pattern of drug release and suitable for oral administration that is equally useful for the delivery of any form of eletriptan, regardless of its solubility. It has now been unexpectedly and advantageously found that when eletriptan or a pharmaceutically acceptable salt thereof is formulated as a core of drug, coated with a certain water-insoluble, polymeric membrane, such a formulation is capable of achieving a sigmoidal pattern of controlled drug release when administered orally, in spite of the unpredictability of such technology and the variable, sometimes low solubility of the different salt forms.

Accordingly, the invention provides a pharmaceutical composition in particulate form, suitable for oral administration, including a core containing eletriptan hydrobromide the core being coated with a water-insoluble, permeable coating consisting of one or more acrylic copolymer(s) containing trimethylammoniumethylmethacrylate groups and, optionally, one or more of a plasticiser, an anti-tacking agent or a wetting agent, said composition being capable of achieving a sigmoidal pattern of controlled drug release.

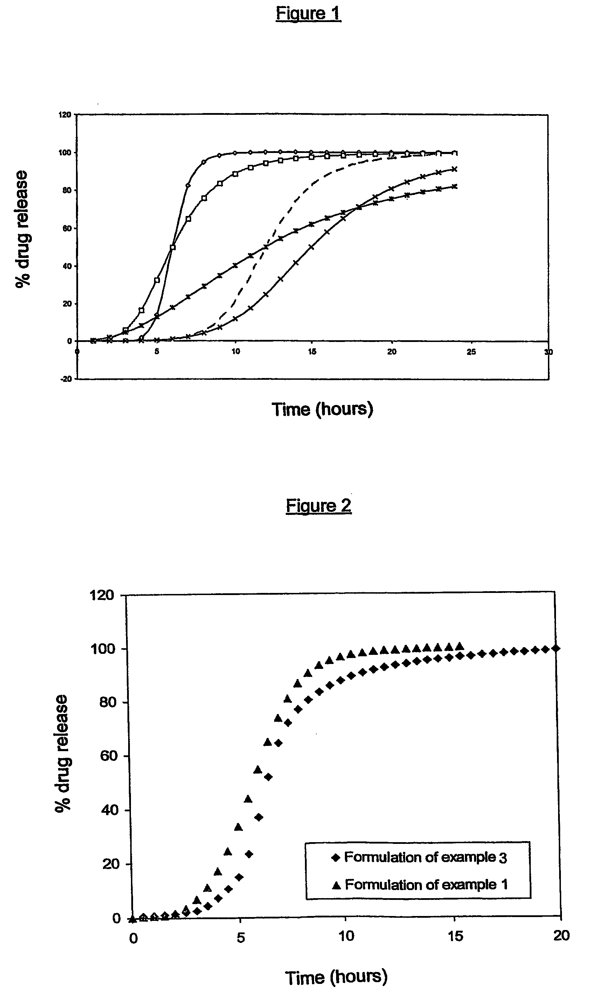

The term 'controlled drug release' means control of the rate of dissolution of the drug in a body fluid (e.g. in the gastrointestinal tract) such that it is slower than the intrinsic dissolution rate of the drug in such a medium. It may otherwise or additionally mean a delayed release of drug. In all these cases, such controlled release is brought about by the nature of the drug's formulation. This effect results in the drug being released into solution over a longer period than would be achieved if the drug was administered without control of its release pattern and/or after an initial delay. The pattern of controlled drug release achieved by the present formulation is known as sigmoidal, i.e. a release profile exhibiting (a) an optional lag time from administration during which no drug or very little drug (e.g. less than 5% by weight) is released, followed by (b) a phase where the rate of drug release increases, followed by (c) a phase where the rate of drug release decreases towards zero as the amount of drug in the formulation is exhausted. The changeover from phase (b) to phase (c) usually occurs when at least 50% by weight of the drug has been released. Specific examples of sigmoidal controlled drug release profiles are given in Example 6, for Examples of the present invention. Other patterns of sigmoidal controlled drug release are illustrated by Figure 1.

In a further aspect, the invention provides a particulate composition, as defined above, for use as a medicament, especially in (a) the treatment of a disease for which a 5-HT_{1B/1D} receptor agonist is indicated, (b) the treatment of migraine or (c) the prevention of migraine recurrence.

In a further aspect, the invention provides the use of a particulate composition as defined above in the manufacture of a medicament for (a) the treatment of migraine or (b) the prevention of migraine recurrence.

A pharmaceutically acceptable hydrobromic acid addition salt of etetriptan may be readily prepared by mixing together solutions of eletriptan and hydrobromic acid. The salt may precipitate from solution and be collected by filtration or may be recovered by evaporation of the solvent.

Also included within the scope of the present invention are polymorphs and solvates (including hydrates) of eletriptan hydrobromide.

The core of the particulate composition, which preferably does not contain an organic acid, may be constituted in several ways. For example, in one embodiment, eletriptan hydrobromide is optionally combined with one or more pharmaceutically acceptable extrusion aid(s) (e.g. a microcrystalline cellulose, a microcrystalline collagen, an amylose, pregelled starch, bentonite or a pharmaceutically acceptable clay such as kaolin), binder(s) (e.g. a polyvinyl pyrrolidone, a copolymer of vinyl pyrrolidone/vinyl acetate, a hydroxypropyl methylcellulose or sodium carboxymethylcellulose) or diluent(s) (e.g. lactose, mannitol or sucrose) and formed into particles suitable for coating (for instance, by extrusion spheronisation, direct pellitisation/high or low shear granulation, fluid bed granulation or spray drying/melt congealing) to form the drug core. In another embodiment, eletriptan hydrobromide optionally in combination with a pharmaceutically acceptable binder (e.g. a hydroxypropyl methylcellulose, a hydroxypropyl cellulose, acacia, carboxymethylcellulose sodium, dextrin, ethylcellulose, gelatin, glucose, guar gum, hydroxyethyl cellulose, methylcellulose, a polymethacrylate, a polyvinyl pyrrolidone, pregelatinised starch, sodium alginate or zein) is layered onto the surface of a pharmaceutically acceptable seed, typically a particle (e.g. a sphere) of sucrose, starch, microcrystalline cellulose or any combination thereof, to form the drug core. Such layering may be by solution layering (in a suitable solvent such as water or a mixture of water and ethanol) or powder layering. The embodiment preferred will depend on the particular form of drug used. For example, with drug forms which have adequate solubility in suitable solvents, such as eletriptan hemisulphate, layering a solution of the drug onto a pharmaceutically acceptable seed may be preferred.

Such a pharmaceutically acceptable seed is preferably a non-pareil sugar/starch sphere of 18-20 mesh, 25-30 mesh or 35-40 mesh or a Celphere CP-507 microcrystalline starch sphere and is most preferably a non-pareil sugar/starch sphere of 25-30 mesh.

The core typically has a width or diameter of from 0.2 to 2 mm, preferably of from 0.5 to 1.4 mm. The amount of eletriptan present in the core will typically be from 10 to 90% by weight, preferably from 20 to 60% by weight, most preferably from 40 to 60% by weight.

In order to provide a smooth surface to the core, to prevent attrition of the core during later stages of manufacture, or to prevent diffusion of eletriptan or a pharmaceutically acceptable salt thereof out of the core during the manufacture or storage of the composition, an additional protective layer, typically composed of a hydroxypropyl methylcellulose, a hydroxypropyl cellulose, a poly(vinyl alcohol), another hydrophilic polymer, or any mixture thereof, is preferably inserted between the core and the water-insoluble, permeable coating. The protective layer preferably comprises a hydroxypropyl methylcellulose. Typically, protective coating levels will vary from 1 to 10% by weight, preferably from 1 to 3% by weight.

The core may also optionally comprise an antioxidant, e.g. ascorbic acid or citric acid.

The term 'water-insoluble, permeable coating' used in the definition of the particulate composition above means a coating which is resistant to degradation under the aqueous conditions encountered in the gastrointestinal tract for at least 24 hours but which is permeable to eletriptan, or a pharmaceutically acceptable salt thereof, when in contact with an aqueous medium so as to allow the passage of dissolved drug through the coating. The rate of drug release will vary with coating thickness which is typically from 10 to 100 microns, preferably from 20 to 50 or from 40 to 80 microns.

The acrylic copolymer(s) containing trimethylammoniumethylmethacrylate groups included in the water-insoluble, permeable coating is/are preferably selected from the Eudragit RL (Trade Mark) and Eudragit RS (Trade Mark) copolymers manufactured by Röhm Pharma GmbH. These copolymers contain chloride counter-ions, which are preferred counter-ions for the present invention. A ratio of about 95:5, by weight, Eudragit RS (Trade Mark):Eudragit RL (Trade Mark) is particularly preferred.

The water-insoluble, permeable coating may include one or more additional substances other than an acrylic copolymer containing trimethylammoniumethylmethacrylate groups, such as a plasticiser (e.g. an acetylated monoglyceride, triethyl citrate, acetyltriethyl citrate, tributyl citrate, acetyltributyl citrate, another citrate ester, dibutyl phthalate, diethyl phthalate, another phthalate ester, diethyl sebacate, dibutyl sebacate, diethyl fumarate, diethyl succinate, a polyethylene glycol, glycerol, sesame oil, a lanolin alcohol or triacetin), an anti-tacking agent (e.g. talc, calcium stearate, colloidal silicon dioxide, glycerin, magnesium stearate, mineral oil, a polyethylene glycol, zinc stearate, aluminium stearate or glycerol monostearate) or a wetting agent (e.g. sodium lauryl sulphate, stearyl alcohol, acacia, benzalkonium chloride, cetomacrogol emulsifying wax, cetostearyl alcohol, cetyl alcohol, cholesterol, diethanolamine, sodium stearate, glycerol monostearate, hydroxypropylcellulose, a lanolin alcohol, triethanolamine, lecithin, poloxamer, a polyoxyethylene alkyl ether, a sorbitan ester, a stearyl alcohol or simethicone). When used, the preferred plasticiser is triethylcitrate, the preferred anti-tacking agent is talc and the preferred wetting agent is sodium lauryl sulphate. The amount of plasticiser used is preferably from 0-30% by weight compared with the amount of acrylic copolymer used and is most preferably about 20%. The amount of anti-tacking agent used is preferably from 0-150% by weight compared with the amount of acrylic copolymer used and is most preferably 50-100%. The amount of wetting agent used is preferably from 0-5% by weight compared with the amount of acrylic copolymer used.

The particulate composition may optionally be further coated with a hydrophilic polymer to provide a smooth surface, to prevent attrition of during later stages of manufacture or to colour the bead. The further coating is typically composed of a hydroxypropyl methylcellulose, a hydroxypropyl cellulose, a poly(vinylalcohol) or any combination thereof and may also contain a dye or pigment. Such a further coating preferably comprises a hydroxypropyl methylcellulose. When present this further coat will typically account for from 1 to 10% by weight of the final product, preferably from 1 to 3%.

The preferred profile of sigmoidal drug release according to the present invention can be defined by the following ranges, which refer to the amount of drug released into a phosphate buffer of pH 7.5 (see the European Pharmacopeia for preparation), containing sodium chloride at a concentration of 0.1 mol/l. The profiles were measured using a dissolution apparatus type 1 (baskets) according to USP XXIV, at 100 rpm and 37 °C.

| Amount drug released (% by weight) | Time (hours) |
|---|---|
| 5 | 1.5-12 |
| 50 | 5.0-15 |
| 80 | 6.5-20 |

Such a particulate composition for achieving a sigmoidal pattern of controlled drug release, as described above, may be combined with a composition of the drug that achieves an immediate release to provide a dual release formulation. The overall release of drug from such a dual release formulation in the gastrointestinal tract will then be characterised by (a) a rapid release of drug on administration of the dosage form that quickly peaks and falls back to near zero (b) an optional lag time during which no drug or very little drug is released (c) a phase where the rate of drug release increases again and (c) a phase where the rate of drug release decreases towards zero as the amount of drug in the formulation is exhausted. Phase (a) can be attributed to the immediate release composition and phases (b)-(d) to the sigmoidal controlled release composition. Such a dual release formulation is particularly useful in treating a patient for both migraine and the prevention of migraine recurrence with a single dose of drug.

Thus in a further aspect, the invention provides a dual release formulation which comprises a sigmoidal controlled release particulate composition of eletriptan hydrobromide as defined above, in combination with an immediate release composition of eletriptan, or a pharmaceutically acceptable salt thereof.

In a further aspect, the invention provides the use of dual release formulation, as defined above, in the manufacture of a medicament for the treatment of migraine and the prevention of migraine recurrence.

In a further aspect, the invention provides a dual release formulation, as defined above, for use in the treatment of migraine and the prevention of migraine recurrence.

A composition that would achieve a more immediate release of eletriptan is the core of the particulate composition described above alone, i.e. without the water-insoluble permeable coating, optionally additionally comprising a disintegrant such as a sodium cross-linked carboxymethylcellulose.

Different particulate compositions according to the invention may also be mixed together to provide formulations with composite release profiles which in certain cases may be zero order.

A particulate composition of the invention can be administered alone or in admixture with a suitable pharmaceutical excipient, diluent or carrier selected with regard to the intended route of administration and standard pharmaceutical practice.

Thus in a further aspect, the invention provides a pharmaceutical formulation including a particulate composition of the invention and one or more pharmaceutically acceptable excipient(s), diluent(s) or carrier(s).

A particulate formulation of the invention is preferably administered orally in the form of tablets, capsules or ovules, which may contain flavouring or colouring agents.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Such capsules may be made of hard or soft gelatine or hydroxypropyl methylcellulose and contain excipients such as lactose, starch, a cellulose, milk sugar or a high molecular weight polyethylene glycol.

The particulate compositions of the invention are most preferably administered contained in hard gelatine capsules.

The daily dosage level of eletriptan or a pharmaceutically acceptable salt thereof will usually be from 1 to 4 mg/kg (in single or divided doses).

Thus tablets or capsules comprising the particulate formulations of the invention will typically contain from 20 to 240 mg of eletriptan hydrobromide for administration singly or two or more at a time, as appropriate. The physician will in any event determine the actual dosage which will be most suitable for any individual patent. The above dosages are exemplary of the average case. There can, of course, be individual instances where higher or lower dosage ranges are merited and such are within the scope of this invention.

The particulate compositions of the invention can also be administered in combination with a prokinetic or antiemetic agent such as metoclopramide (for example, see WO-A-00/25778).

It is to be understood that all references herein to treatment include curative, palliative and prophylactic treatment.

The following Examples illustrate the invention.

### Example 1: Particulate composition containing eletriptan hydrobromide

### Preparation of drug-containing core:

A dry blend is made up by mixing 1455.0g eletriptan hydrobromide, 773.0g of microcrystalline cellulose (Avicel PH101) and 773.0g lactose in a planetary mixer (EG20, Peerless). Purified water (1400g) is added to give a wet mass that is subsequently extruded using a 1.0 mm screen (Nica extruder E140, Aeromatic Fielder). The extrudates are rounded in a spheroniser (Caleva Model 15) and thoroughly dried in a fan-assisted oven at 40 °C for 12 hours. The extrudates have the following approximate size distribution, referring to their diameter: <0.71 mm, 8% (by weight); 0.71-1.18mm, 89.5% (by weight); 1.18-1.4mm, 2% (by weight); >1.4mm, 0.5% (by weight). The 0.71-1.18mm fraction is used in the subsequent coating step.

### Preparation of coating dispersion

A coating dispersion is made in the following manner. In a container equipped with a mixer, 20.0g talc is added to 331.7g purified water to make up a talc dispersion. Subsequently, 8.0g triethyl citrate (TEC) followed by 126.7g Eudragit (trade mark) RS30D (30 % w/w solids content) and 6.7g Eudragit (trade mark) RL30D (30 % w/w solids content) are added. The coating dispersion is mixed thoroughly.

### Coating procedure

The drug-containing cores (500g) are dispensed into the Wurster fluid bed coater (Strea-1, Aeromatic Fielder). The coating dispersion is applied until it is depleted. When the coat application is completed, the product is dried under the same conditions for five minutes and then discharged. The following approximate size distribution, referring to diameter, is obtained: <0.71mm, 2.6% (by weight); 0.71-1.18mm, 97.3% (by weight); 1.18-1.4mm, 0.1% (by weight); >1.4mm, 0% (by weight). The particles are dusted with 28.4g talc to prevent them from sticking during the curing step. The particles are cured in a fan-assisted oven at 40 °C for 24 hours to complete the membrane-forming process and to remove excess moisture. Excess talc is removed by screening through an appropriate size mesh.

### Example 2: Particulate composition containing eletriptan hemisulphate

This example does not fall within the scope of the claims.

The following 'particulate composition of eletriptan hemisulphate is designed to have a release rate such that half of the drug content is released in the first 12 hours following oral administration.

### Preparation of drug-layered core

A drug layer solution is prepared by adding 640g purified water to a container equipped with a mixer. With vigorous mixing, 26.7g hydroxypropyl methylcellulose (Methocel E50LV), 2.7g polyethylene glycol (PEG 400) and 133.3g eletriptan hemisulphate are dissolved therein. Mixing is continued until complete dissolution is achieved. Finally 426.7g ethanol is added and the solution is mixed thoroughly.

Non-pareil seeds (134.8g, 18/20 mesh, Nu-Pareil) are dispensed into a Wurster fluid bed coater (Strea-1, Aeromatic Fielder). After fluidisation of the non-pareils, spraying of the drug layer solution is commenced to layer drug solution effectively onto the seeds. Spraying is continued until the drug layer solution is exhausted. The beads are dried under the same conditions for 15 minutes. The beads have the following approximate size distribution, referring to their diameter: <1.18mm, 9.9% (by weight); 1.18-1.4mm, 70.5% (by weight); 1.4-1.7mm, 14.6% (by weight); >1.7mm, 5% (by weight). The 1.18-1.4mm fraction is used in the subsequent coating step.

### Preparation of coating dispersion

A coating dispersion is made in the following manner. In a container equipped with a mixer, 22.5g talc is added to 292.5g purified water to make up a talc dispersion. Subsequently, 9.0g triethyl citrate (TEC) followed by 142.5g Eudragit (trade mark) RS30D (30 % w/w solids content) and 7.5g Eudragit (trade mark) RL30D (30 % w/w solids content) are added. The coating dispersion is mixed thoroughly.

### Coating procedure

The drug-containing cores (200g) are dispensed into a Wurster film coater (Strea-1, Aeromatic Fielder). The coating dispersion is applied until it is depleted. When the coat application is completed, the product is dried under the same conditions for five minutes and then discharged. The particles so obtained have the following approximate size distribution, referring to their diameter: <1.18mm, 8% (by weight); 1.18-1.4mm, 56% (by weight); 1.4-1.7mm, 30% (by weight); >1.7mm, 6% (by weight). The particles of the 1.18-1.4mm fraction are dusted with 12.5g talc to prevent them from sticking during curing. The particles are cured in a fan-assisted oven at 40 °C for 24 hours to complete the membrane formation process and to remove excess moisture. Excess talc is removed by screening using an appropriate size mesh.

### Example 3: Particulate composition containing eletriptan hemisulphate

This example does not fall within the scope of the claims.

The following particulate composition of eletnptan hemisulphate is designed to have a release rate such that half of the drug content is released in the first 7 hours following oral administration.

### Preparation of coating dispersion

A coating dispersion is made in the following manner. In a container equipped with a mixer, 30.0g talc is added to 390.0g purified water to make up a talc dispersion. Subsequently, 12.0g triethyl citrate (TEC) followed by 190.0g Eudragit (trade mark) RS30D (30 % w/w solids content) and 10.0g Eudragit (trade mark) RL30D (30 % w/w solids content) are added. The coating dispersion is mixed thoroughly.

### Coating procedure

Drug-containing cores (400g), prepared by the method of Example 2, are dispensed into a Wurster film coater (Strea-1, Aeromatic Fielder). The coating dispersion is applied until it is depleted. When the coat application is completed, the product is dried under the same conditions for five minutes and then discharged. The particles are dusted with 50g talc to prevent them from sticking during curing. The particles are cured in a fan-assisted oven at 40 °C for 24 hours to complete the membrane formation process and to remove excess moisture. Excess talc is removed by screening using an appropriate size mesh.

### Example 4: Particulate composition containing eletriptan hemisulphate

This example does not fall within the scope of the claims.

### Preparation of drug-layered core

A drug layer solution is prepared by dissolving eletriptan hemisulphate (1017.8g) in purified water (1690g) with mixing. Talc (203.6g) is then dispersed in the solution.

Non-pareil seeds (1000g, 25/30 mesh, Nu-Pareil) are dispensed into a Wurster fluid bed coater (GPCG-1, Glatt). After fluidisation of the non-pareils, spraying of the drug layer solution is commenced to layer drug solution effectively onto the seeds. Spraying is continued until the drug layer solution is exhausted. A solution of hydroxypropyl methylcellulose [Opadry Orange (Trade Mark) 2 (55.3g)] in purified water (405.4g) is then sprayed onto the seeds. The beads are dried under the same conditions for 5 minutes.

### Preparation of coating dispersion

A coating dispersion is made in the following manner. In a container equipped with a mixer, 182.8g talc is added to 1216g purified water to make up a talc dispersion. Subsequently, 73.2g triethyl citrate (TEC) followed by 1157.7g Eudragit (trade mark) RS30D (30 % w/w solids content) and 60.9g Eudragit (trade mark) RL30D (30 % w/w solids content) are added. The coating dispersion is mixed thoroughly.

### Coating procedure

The drug-containing cores (1000g) are dispensed into a Wurster film coater (GPCG-1, Glatt). The coating dispersion is applied until it is depleted. A solution of hydroxypropyl methylcellulose [Opadry Blue (Trade Mark) 2 (40g)] in purified water (293g) is then sprayed onto the cores. When the coat application is completed, the product is dried under the same conditions for five minutes and then discharged. The particles so obtained have an approximate size distribution, referring to their diameter, of 1.0-1.18mm. The particles are cured in a fan-assisted oven at 40 °C for 24 hours to complete the membrane formation process and to remove excess moisture.

### Example 5: Immediate release formulation of eletriptan hemisulphate

A drug layer solution is prepared by adding 640g purified water to a container equipped with a mixer. With vigorous mixing, 26.7g hydroxypropyl methylcellulose (Methocel E50LV), 2.7g polyethylene glycol (PEG 400) and 133.3g eletriptan hemisulphate are dissolved therein. Mixing is continued until complete dissolution is achieved. Finally 426.7g ethanol is added and the solution is mixed thoroughly.

Non-pareil seeds (134.8g, 18/20 mesh, Nu-Pareil) are dispensed into a Wurster fluid bed coater (Strea-1, Aeromatic Fielder). After fluidisation of the non-pareils, spraying of the drug layer solution is commenced to layer drug solution effectively onto the seeds. Spraying is continued until the drug layer solution is exhausted. The beads are dried under the same conditions for 15 minutes. The beads have the following approximate size distribution, referring to their diameter: <1.18mm, 9.9% (by weight); 1.18-1.4mm, 70.5% (by weight); 1.4-1.7mm, 14.6% (by weight); >1.7mm, 5% (by weight). The 1.18-1.4mm fraction is retained for use.

### Example 6: Determination of the in vitro drug release profiles of Examples 1 and 3

Sigmoidal controlled drug release is illustrated by Figure 2 which plots the rate of release of eletriptan hydrobromide and eletriptan hemisulphate from separate particulate compositions of the present invention into water buffered to pH 7.5 (see the European Pharmacopeia) containing 0.1 mol/l of sodium chloride. The particulate compositions used are the two formulations described in Examples 1 and 3 respectively. A dissolution type 1 apparatus with baskets was used according to USP XXIV at 100 rpm and 37 °C.

For the formulation of Example 1, the lag time during which up to 5% by weight of the drug was released was about 2.5 hours, 50% by weight of the drug was released by about 5.75 hours and 95% by weight of the drug was released by about 10 hours. For the formulation of Example 3, the lag time during which up to 5% by weight of the drug was release was about 3.5 hours, 50% by weight of the drug was released by about 6.5 hours and 95% by weight of the drug was released by about 14.5 hours.

### Example 7 - In vivo drug release profiles of a dual release formulation comprising the composition of Example 2 or Example 3 and the immediate release formulation of Example 5

Twelve healthy volunteers (6 male and 6 female) were each dosed with eletriptan hemisulphate according to the following three treatment regimens A-C in the fasted state.

| | Dose of eletriptan hemisulphate delivered by immediate release formulation (Example 5) | Dose of eletriptan hemisulphate delivered by sigmoidal release formulation |
|---|---|---|
| A | 40mg | - |
| B | 40 mg | 40mg (Example 2) |
| C | 40 mg | 40 mg (Example 3) |

The drug was administered in the form of hard gelatine capsules (size 1). In regimen A the capsule was filled with 100mg of the formulation of Example 5. In regimen B the capsule was filled with 100mg of the formulation of Example 5 and 138mg of the composition of Example 2. In regimen C, the capsule was filled with 100mg of the formulation of Example 5 and 125mg of the composition of Example 3.

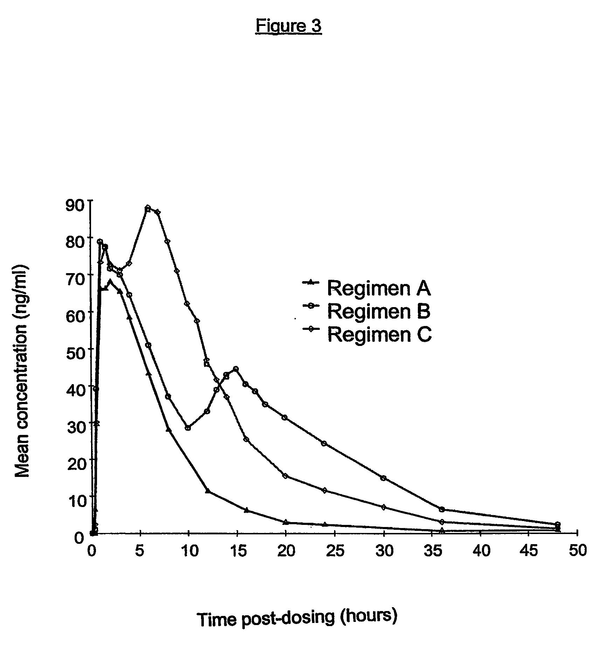

Thus, the volunteers received an immediate release formulation of eletriptan hemisulphate (40mg) in regimen A and dual release formulations containing an immediate release formulation of eletriptan hemisulphate (40mg) and a sigmoidal release formulation of eletriptan hemisulphate (40mg) in regimens B and C. The regimens were spaced such that at least 7 days was allowed between drug dosing. Blood samples were collected at spaced time points for a period of 48 hours following dosing in each case and the samples were analysed for eletriptan. The results are shown in Figure 3 which plots the mean plasma concentration of eletriptan across the 12 volunteers against time from dosing.

The dual release formulations of eletriptan hemisulphate all delivered a mean plasma concentration of greater than 10 ng/ml at 20 hours post-dosing whilst providing a mean peak plasma concentration of less than 100 ng/ml within the first 10 hours post-dosing.

### FIGURES

In the Figures that follow:
Figure 1 shows representative patterns of sigmoidal controlled drug release;
Figure 2 shows a pattern of sigmoidal controlled drug release achieved by the compositions of each of Examples 1 and 3; and
Figure 3 shows the *in vivo* drug release profile of a dual release formulation comprising the composition of Example 2 or Example 3 (see Example 7).

## Claims

1. A pharmaceutical composition in particulate form, suitable for oral administration, including a core containing eletriptan hydrobromide, the core being coated with a water-insoluble, permeable coating consisting of one or more acrylic copolymer(s) containing trimethylammoniumethylmethacrylate groups and, optionally, one or more of a plasticiser, an anti-tacking agent or a wetting agent, said composition being capable of achieving a sigmoidal pattern of controlled drug release.

2. A composition, as claimed in claim 1, wherein the core has a diameter of from 0.5 to 1.4mm.

3. A composition, as claimed in claim 1 or claim 2, wherein the core contains from 40 to 60% w/w of eletriptan.

4. A composition, as claimed in claim 1 wherein the core includes eletriptan hydrobromide, microcrystalline cellulose and lactose.

5. A composition, as claimed in any preceding claim, wherein an additional protective layer is inserted between the core and the water-insoluble, permeable coating.

6. A composition, as claimed in claim 5; wherein the additional protective layer includes a hydroxypropyl methylcellulose.

7. A composition, as claimed in any preceding claim, wherein the acrylic copolymer(s) containing trimethylammoniumethylmethacrylate groups is/are selected from Eudragit RL (Trade Mark) and Eudragit RS (Trade Mark).

8. A composition, as claimed in claim 7, wherein the acrylic copolymers are a mixture of 95:5, by weight, Eudragit RS (Trade Mark):Eudragit RL (Trade Mark).

9. A composition, as claimed in any preceding claim, wherein the water-insoluble, permeable coating has a thickness of from 40 to 80 microns.

10. A composition, as claimed in claim 7, wherein the water-insoluble, permeable coating includes Eudragit RL (Trade Mark), Eudragit RS (Trade Mark), talc and triethyl citrate.

11. A pharmaceutical formulation including a composition, as defined in any preceding claim, and one or more pharmaceutically acceptable excipient(s), diluent(s) or carrier(s).

12. A pharmaceutical formulation, as claimed in claim 11, which is a hard gelatine capsule.

13. A dual release formulation which includes a sigmoidal controlled release composition, as claimed in any one of claims 1 to 10, in combination with an immediate release composition of eletriptan, or a pharmaceutically acceptable salt thereof.

14. A composition as claimed in any one of claims 1 to 10 or a formulation as claimed in any one of claims 11 to 13 for use as a medicament.

15. The use of a composition as claimed in any one of claims 1 to 10 or a formulation as claimed in any one of claims 11 to 13 in the manufacture of a medicament for (a) the treatment of migraine or (b) the prevention of migraine recurrence.

16. The use of dual release formulation as claimed in claim 13 in the manufacture of a medicament for the treatment of migraine and the prevention of migraine recurrence.

17. A process for the preparation of a particulate composition, as claimed in claim 1 comprising (a) forming a core containing eletriptan hydrobromide and (b) coating the core with a water-insoluble, permeable coating consisting of one or more acrylic copolymer(s) containing trimethylammoniumethylmethacrylate groups and, optionally, one or more of a plasticiser, an anti-tacking agent or a wetting agent.

18. A process for the preparation of a particulate composition, as claimed in claim 1 comprising (a) forming a core by layering eletriptan hydrobromide and, optionally, a pharmaceutically acceptable binder onto the surface of a pharmaceutically acceptable seed and (b) coating the core with a water-insoluble, permeable coating consisting of one or more acrylic copolymer(s) containing trimethylammoniumethylmethacrylate groups and, optionally, one or more of a plasticiser, an anti-tacking agent or a wetting agent.

## Patentansprüche

1. Partikuläre, zur oralen Verabreichung geeignete pharmazeutische Zusammensetzung, enthaltend einen Eletriptan-Hydrobromid enthaltenden Kern, der mit einem wasserunlöslichen, durchlässigen Überzug versehen ist, der aus einem oder mehreren Trimethylammoniumethylmethacrylat-Gruppen enthaltenden Acryl-Copolymeren und optional aus einem oder mehreren Weichmachern, Trennmitteln oder Feuchtmitteln besteht, wobei die Zusammensetzung ein sigmoides Freisetzungsmuster gewährleisten kann.

2. Zusammensetzung nach Anspruch 1, in der der Kern einen Durchmesser von 0,5 bis 1,4 mm aufweist.

3. Zusammensetzung nach Anspruch 1 oder 2, in der der Kern 40 - 60 Gew.% Eletriptan enthält.

4. Zusammensetzung nach Anspruch 1, in der der Kern Eletriptan-Hydrobromid, mikrokristalline Cellulose und Lactose enthält.

5. Zusammensetzung nach einem der vorhergehenden Ansprüche, in der zwischen dem Kern und dem wasserunlöslichen, durchlässigen Überzug eine zusätzliche Schutzschicht angeordnet ist.

6. Zusammensetzung nach Anspruch 5, in der die zusätzliche Schutzschicht Hydroxypropylmethylcellulose enthält.

7. Zusammensetzung nach einem der vorhergehenden Ansprüche, in der das (die) Trimethylammoniumethylmethycrylat-Gruppen enthaltende(n) AcrylCopolymere(n) ausgewählt ist (sind) aus Eudragit RL™ und Eudragit RS™.

8. Zusammensetzung nach Anspruch 7, in der die Acryl-Copolymeren ein 95 : 5 Gew.%-Gemisch aus Eudragit RS™ und Eudragit RS™ darstellen.

9. Zusammensetzung nach einem der vorhergehenden Ansprüche, in der der wasserunlösliche, durchlässige Überzug eine Stärke von 40 - 80 µ aufweist.

10. Zusammensetzung nach Anspruch 7, in der der wasserunlösliche, durchlässige Überzug Eudragit RL™, Eudragit RS™, Talkum und Triethylcitrat enthält.

11. Pharmazeutische Formulierung, enthaltend eine Zusammensetzung nach einem der vorhergehenden Ansprüche und einen oder mehrere pharmazeutisch akzeptable Excipienten, Verdünner oder Trägerstoffe.

12. Pharmazeutische Formulierung nach Anspruch 11, die eine Hartgelatine-Kapsel darstellt.

13. Dual-Release-Formulierung, enthaltend eine Zusammensetzung mit sigmoidem Freisetzungsmuster nach einem der Ansprüche 1 - 10 in Kombination mit einer Eletriptan oder dessen pharmazeutisch akzeptables Salz enthaltenden Immediate-Release-Zusammensetzung.

14. Zusammensetzung nach einem der Ansprüche 1 - 10 oder Formulierung nach einem der Ansprüche 11 - 13 als Arzneimittel.

15. Verwendung einer Zusammensetzung nach einem der Ansprüche 1 - 10 oder einer Formulierung nach einem der Ansprüche 11 - 13 zur Herstellung eines Arzneimittels zur (a) Behandlung von Migräne oder (b) Prävention des Wiederauftretens von Migräne.

16. Verwendung der Dual-Release-Formulierung nach Anspruch 13 zur Herstellung eines Arzneimittels zur Behandlung von Migräne und zur Prävention des Wiederauftretens von Migräne.

17. Verfahren zur Herstellung einer partikulären Zusammensetzung nach Anspruch 1, umfassend (a) die Bildung eines Eletriptan-Hydrobromid enthaltenden Kerns und (b) das Überziehen des Kerns mit einem wasserunlöslichen, durchlässigen Überzug, der aus einem oder mehreren Trimethylammoniumethylmethycrylat-Gruppen enthaltenden Acryl-Copolymeren und optional aus einem oder mehreren Weichmachern, Trennmitteln oder Feuchtmitteln besteht.

18. Verfahren zur Herstellung einer partikulären Zusammensetzung nach Anspruch 1, umfassend (a) die Bildung eines Kerns durch Aufbringen (layering) von Eletriptan-Hydrobromid und optional eines pharmazeutisch akzeptablen Bindemittels auf die Oberfläche eines pharmazeutisch akzeptablen Keims und (b) das Überziehen des Kerns mit einem wasserunlöslichen, durchlässigen Überzug, der aus einem oder mehreren Trimethylammoniumethylmethycrylat-Gruppen enthaltenden Acryl-Copolymeren und optional einem oder mehreren Weichmachern, Trennmitteln oder Feuchtmitteln besteht.

## Revendications

1. Composition pharmaceutique sous forme de particules, convenable pour l'administration orale, comprenant un noyau contenant du bromhydrate d'élétriptan, le noyau étant enrobé avec un enrobage perméable, insoluble dans l'eau, consistant en un ou plusieurs copolymères acryliques et, facultativement, un ou plusieurs des agents consistant en un plastifiant, un agent anti-adhésivité ou un agent mouillant, ladite composition étant capable de présenter un schéma sigmoide de libération contrôlée de médicament.

2. Composition suivant la revendication 1, dans laquelle le noyau a un diamètre de 0,5 à 1,4 mm.

3. Composition suivant la revendication 1 ou la revendication 2, dans laquelle le noyau contient 40 à 60 % en poids/poids d'élétriptan.

4. Composition suivant la revendication 1, dans laquelle le noyau comprend du bromhydrate d'élétriptan, de la cellulose microcristalline et du lactose.

5. Composition suivant l'une quelconque des revendications précédentes, dans laquelle une couche protectrice supplémentaire est insérée entre le noyau et l'enrobage perméable insoluble dans l'eau.

6. Composition suivant la revendication 5, dans laquelle la couche protectrice supplémentaire comprend une hydroxypropylméthylcellulose.

7. Composition suivant l'une quelconque des revendications précédentes, dans laquelle le ou les copolymères acryliques contenant des groupes triméthylammoniuméthylméthacrylate sont choisis entre l'Eudragit RL (marque commerciale) et l'Eudragit RS (marque commerciale).

8. Composition suivant la revendication 7, dans laquelle les copolymères acryliques consistent en un mélange dans le rapport pondéral 95:5 d'Eudragit RS (marque commerciale) : Eudragit RL (marque commerciale).

9. Composition suivant l'une quelconque des revendications précédentes, dans laquelle l'enrobage perméable insoluble dans l'eau a une épaisseur de 40 à 80 micromètres.

10. Composition suivant la revendication 7, dans laquelle l'enrobage perméable insoluble dans l'eau comprend de l'Eudragit RL (marque commerciale) et de l'Eudragit RS (marque commerciale), du talc et du citrate de thriéthyle.

11. Formulation pharmaceutique comprenant une composition, répondant à la définition suivant l'une quelconque des revendications précédentes, et un ou plusieurs excipients, diluants ou supports pharmaceutiquement acceptables.

12. Formulation pharmaceutique suivant la revendication 11, qui est une gélule.

13. Formulation à libération double, qui comprend une composition à libération contrôlée sigmoide, suivant l'une quelconque des revendications 1 à 10, en association avec une composition à libération immédiate d'élétriptan ou d'un de ses sels pharmaceutiquement acceptables.

14. Composition suivant l'une quelconque des revendications 1 à 10, ou formulation suivant l'une quelconque des revendications 11 à 13, destinée à être utilisée comme médicament.

15. Utilisation d'une composition suivant l'une quelconque des revendications 1 à 10, ou d'une formulation suivant l'une quelconque des revendications 11 à 13, dans la production d'un médicament pour (a) le traitement de la migraine, ou (b) la prévention de la récurrence de la migraine.

16. Utilisation d'une formulation à libération double suivant la revendication 13, dans la production d'un médicament destiné au traitement de la migraine et à la prévention de la récurrence de la migraine.

17. Procédé pour la préparation d'une composition en particules, suivant la revendication 1, comprenant les étapes consistant (a) à former un noyau contenant de l'élétriptan ou un des ses sels pharmaceutiquement acceptables, et (b) à enrober le noyau avec un enrobage perméable, insoluble dans l'eau, comprenant un ou plusieurs copolymères acryliques contenant des groupes triméthylammoniuméthylméthacrylate et, facultativement, un ou plusieurs des agents consistant en un plastifiant, un agent anti-adhésivité et un agent mouillant.

18. Procédé pour la préparation d'une composition en particules suivant la revendication 1, comprenant les étapes consistant (a) à former un noyau en déposant en couche du bromhydrate d'élétriptan et, facultativement, un liant pharmaceutiquement acceptable sur la surface d'un germe pharmaceutiquement acceptable, et (b) à enrober le noyau avec un enrobage perméable, insoluble dans l'eau, comprenant un ou plusieurs copolymères acryliques contenant des groupes triméthylammoniuméthylméthacrylate et, facultativement, un ou plusieurs des agents consistant en un plastifiant, un agent anti-adhésivité et un agent mouillant.
